# EUROPÄISCHE PATENTSCHRIFT

(11) **EP 2 049 006 B1**
(45) Veröffentlichungstag und Bekanntmachung des Hinweises auf die Patenterteilung: **15.03.2017**
(21) Anmeldenummer: 07786481.7
(22) Anmeldetag: 01.08.2007
(51) Int. Cl.: A61B 3/103

(54) **VERFAHREN ZUR BESTIMMUNG VON KORREKTUREIGENSCHAFTEN EINER SEHHILFE**
METHOD FOR DETERMINING CORRECTION CHARACTERISTICS FOR A VISION AID
PROCÉDÉ POUR DÉTERMINER DES PROPRIÉTÉS CORRECTRICES D'UN MOYEN D'AIDE À LA VUE

(30) Priorität: 08.08.2006 DE 102006036958
(43) Veröffentlichungstag der Anmeldung: 22.04.2009
(73) Patentinhaber: Carl Zeiss Vision GmbH, 73430 Aalen (DE)
(72) Erfinder: KELCH, Gerhard, 73431 Aalen (DE); KRATZER, Timo, 73434 Aalen (DE)
(74) Vertreter: Schmid, Wolfgang
(86) Internationale Anmeldenummer: PCT/EP2007/006789
(87) Internationale Veröffentlichungsnummer: WO 2008/017404

(56) Entgegenhaltungen:
- WO-A-03/032825
- WO-A-2005/070285
- US-A1- 2006 023 162

## Beschreibung

Die Erfindung betrifft ein Verfahren zur Bestimmung von Korrektureigenschaften einer Sehhilfe für wenigstens ein Auge einer Person.

Bekannte Verfahren zur Berechnung der Eigenschaften von Sehhilfen bzw. Brillengläsern gehen rein mathematisch/optisch vor. Dabei wird eine Minimierung der Fehler zu einem Betrag von Null durchgeführt, wobei die individuelle Physiologie des Brillenträgers bei der Verarbeitung des Netzhautbildes nicht berücksichtigt wird. Demzufolge entsprechen die ermittelten Eigenschaften der Brillengläser nicht unbedingt den vom Brillenträger gewünschten Eigenschaften hinsichtlich der Sehqualität.

Die US 6,511,180 B2 betrifft ein Verfahren und ein System zur Bestimmung einer refraktiven Korrektur für ein menschliches Auge, mit einer objektiven Messung von Wellenfrontfehlern höherer Ordnung und einer genauen Schätzung der subjektiven Refraktion.

Die WO 02/083078 A2 betrifft ein Verfahren zur Ermittlung einer Augenrefraktion, wobei eine gewünschte Qualität hinsichtlich einer ausgewählten Seheigenschaft erzielt werden soll. Dazu werden Wellenfrontfehler gemessen, um den Status der Augenrefraktion objektiv zu bestimmen. Es wird ausgeführt, dass die Sehqualität ein kumuliertes Ergebnis der Augenrefraktion unter vielen verschiedenen Einsatzbedingungen ist. Die gewünschte Sehqualität kann für verschiedene primäre Anforderungen der einzelnen Patienten unterschiedlich sein. Diese verschiedenen Anforderungen können sich zum Beispiel auf die Nachtsicht beziehen. Objektive und subjektive Refraktionsmessungen werden an Probanden durchgeführt, um Korrelationen zu ermitteln, welche später bei neuen Patienten angewendet werden. Es erfolgt keine individuelle Messung. Zur Erreichung einer gewünschten Sehqualität wird eine Refraktion gemessen und diese mit einer mathematischen Funktion beschrieben. Danach wird eine Optimierung dieser mathematischen Funktion durchgeführt, um die vorgegebene Seheigenschaft im Rahmen der Sehqualität zu verbessern.

US 2006/0023162 offenbart eine Messung von Wellenfrontfehler in unterschiedlichen Bedingungen (z.B. im Hellen und im Dunkel) und die Berechnung von Zielkoeffizienten, die bestimmten Bedingungen erfüllen. Dadurch können die Wellefronten von jeder der unterschiedlichen Bedingungen geändert werden.

Der vorliegenden Erfindung liegt die Aufgabe zugrunde, ein Verfahren der eingangs erwähnten Art zu schaffen, welches den individuellen Sehkomfort für den Träger der Sehhilfe verbessert. Diese Aufgabe wird erfindungsgemäß durch Anspruch 1 gelöst.

Das erfindungsgemäße Verfahren berücksichtigt das subjektive Empfinden des Trägers der Sehhilfe bzw. des Brillenträgers, wobei auf einen bestimmten Fehlerbetrag optimiert wird, bei welchem unter physiologischen Aspekten für den jeweiligen Träger ein individuelles Optimum an Sehkomfort erzielt wird. Der Sehkomfort wird dadurch für die Person wesentlich verbessert.

Dies wird für jede einzelne Person, insbesondere für jedes Auge der Person durch eine Optimierung hinsichtlich der Übereinstimmung des Seheindruckes der Person unter zwei Gebrauchsbedingungen, vorzugsweise im Hellen und im Dunkeln, durchgeführt. Es erfolgt zunächst insbesondere eine Messung des Wellenfrontfehlers und der subjektiven Refraktion bzw. eine Brillenausmessung unter der ersten Gebrauchsbedingung (z. B. im Hellen) zur Bestimmung des Seheindruckes der Person. Danach erfolgt unter der zweiten Gebrauchsbedingung (z. B. im Dunkeln) wiederum eine Bestimmung des Seheindruckes einer Person insbesondere durch die Messung des Wellenfrontfehlers, welcher sich von dem Wellenfrontfehler unter der ersten Gebrauchsbedingung in der Regel unterscheidet (beispielsweise unterschiedliche Pupillenöffnung im Hellen / Dunkeln). Dann wird eine Korrektur der Wellenfrontfehler niederer Ordnung unter der zweiten Gebrauchsbedingung derart durchgeführt, dass ein Seheindruck wie unter der ersten Gebrauchsbedingung entsteht. Die Personen-Wellenfront wird sozusagen solange optimiert, bis die Seheindrücke unter den beiden Gebrauchsbedingungen wenigstens annähernd übereinstimmen. Somit erhält die Person einen optimalen Seheindruck im Hellen, wobei sie sich im Dunkeln nicht umstellen muss. Als weitere Gebrauchsbedingung käme auch beispielsweise eine Blendung in heller Sonne in Betracht.

Dazu können verschiedene weitere Metriken als sogenannte Metametriken, vorzugsweise eine Point-Spread-Funktion, ein Kontrastsehen, und/oder ein dynamisches Sehen parallel berücksichtigt werden.

Als Sehhilfe kommen sowohl Brillengläser als auch Kontaktlinsen oder dergleichen in Frage.

Sonach ergibt sich in vorteilhafter Weise ein kompletter Seheindruck, welcher möglichst viele bzw. alle Fehlsichtigkeiten der Augen der Person aufweist. Dieser Gesamtseheindruck kann dann optimiert werden.

Nachfolgend ist anhand der Zeichnung prinzipmäßig ein Ausführungsbeispiel der Erfindung beschrieben.

Es zeigt:
Figur 1 ein stark vereinfachtes Flussdiagramm eines erfindungsgemäßen Verfahrens; und
Figur 2 eine Verdeutlichung der Wirkung des erfindungsgemäßen Verfahrens.

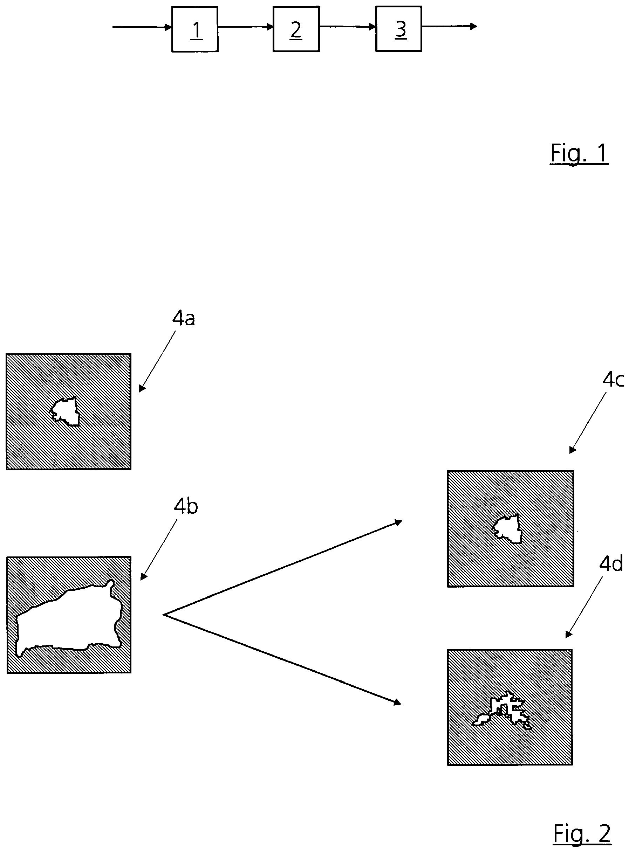

In Fig. 1 ist ein Flussdiagramm eines erfindungsgemäßen Verfahrens zur Bestimmung von Korrektureigenschaften einer Sehhilfe für wenigstens ein Auge einer Person dargestellt, wobei zur Erzielung eines individuellen Optimums an Sehkomfort für die Person eine individuelle Physiologie der Person bei der Verarbeitung des Netzhautbildes berücksichtigt wird.

Dabei wird in einem ersten Schritt 1 ein Seheindruck der Person im Hellen als erste Gebrauchsbedingung mittels einer Wellenfrontfehlermessung und/oder einer Messung einer subjektiven Refraktion bestimmt. Die Wellenfrontfehlermessung erfolgt in bekannter Weise durch eine Vorrichtung, welche sozusagen die Geometrie des Auges ermittelt. Die Ermittlung der subjektiven Refraktion erfolgt durch ein Vorschalten unterschiedlicher Testgläser vor die Augen der Person, wobei ein optimales Testglas ermittelt wird. Aus den Wellenfrontfehlertermen bzw. Zernike-Koeffizienten niederer Ordnung wird die Sphäre-Zylinder-Achse (SCA) zur Korrektur der Sehschärfe (Visus) für die Gläser der Sehhilfe bestimmt.

Danach wird in einem zweiten Schritt 2 ein Seheindruck der Person im Dunkeln als zweite Gebrauchsbedingung durch eine Wellenfrontfehlermessung bestimmt.

In einem dritten Schritt 3 wird die Wellenfront der Person, d. h. die Wellenfrontfehlerterme niederer Ordnung bzw. die Korrektureigenschaften der Sehhilfe solange optimiert, bis der Seheindruck der Person im Dunkeln mit dem Seheindruck der Person im Hellen wenigstens annähernd übereinstimmt.

In Fig. 2 sind Seheindrücke 4a bis 4d, welche über eine Point-Spread-Funktion als Metametrik beschrieben sind, beispielhaft und stark vereinfacht dargestellt. Der Seheindruck 4a ergibt sich im Hellen mit Sehhilfe. Der Seheindruck 4b entsteht im Dunkeln ohne Sehhilfe. Nach Anwendung des erfindungsgemäßen Verfahrens ergibt sich in vorteilhafter Weise ein wenigstens annähernd mit dem Seheindruck 4a übereinstimmender Seheindruck 4c mit Sehhilfe im Dunkeln. Die Person muss sich im Dunkeln somit nicht umstellen. Bei bekannten Verfahren ergibt sich beispielsweise der Seheindruck 4d, welcher nicht mit Seheindruck 4a übereinstimmt, wobei sich die Person in nachteiliger Weise umstellen muss. Dadurch sinkt der Sehkomfort deutlich.

In anderen Ausführungsbeispielen könnten verschiedene weitere Metriken als sogenannte Metametriken, vorzugsweise Kontrastsehen oder dynamisches Sehen parallel berücksichtigt werden.

## Patentansprüche

1. Verfahren zur Bestimmung von Korrektureigenschaften einer Sehhilfe für wenigstens ein Auge einer Person, wobei zur Erzielung eines individuellen Optimums an Sehkomfort für die Person eine individuelle Physiologie der Person bei der Verarbeitung des Netzhautbildes berücksichtigt wird, wobei:
- in einem ersten Schritt (1) ein Seheindruck der Person unter einer ersten Gebrauchsbedingung bestimmt wird, wonach
- in einem zweiten Schritt (2) ein Seheindruck der Person unter einer zweiten Gebrauchsbedingung bestimmt wird, und wonach
- in einem dritten Schritt (3) die Wellenfront der Person durch die Bestimmung der Korrektureigenschaften der Sehhilfe solange optimiert wird, bis der Seheindruck der Person unter der zweiten Gebrauchsbedingung mit dem in dem ersten Schritt (1) bestimmten Seheindruck der Person unter der ersten Gebrauchsbedingung wenigstens annähernd übereinstimmt.

2. Verfahren nach Anspruch 1, **dadurch gekennzeichnet, dass** die erste Gebrauchsbedingung im Hellen ist.

3. Verfahren nach Anspruch 1 oder 2, **dadurch gekennzeichnet, dass** die zweite Gebrauchsbedingung im Dunkeln ist.

4. Verfahren nach Anspruch 1, 2 oder 3, **dadurch gekennzeichnet, dass** parallel weitere Metriken zur Bestimmung des Seheindrucks verwendet werden.

5. Verfahren nach Anspruch 4, **dadurch gekennzeichnet, dass** als weitere Metriken eine Point-Spread-Funktion, ein Kontrastsehen und/oder ein dynamisches Sehen verwendet werden.

6. Verfahren nach einem der Ansprüche 1 bis 5, **dadurch gekennzeichnet, dass** die Sehhilfe ein Brillenglas ist.

7. Verfahren nach einem der Ansprüche 1 bis 6, **dadurch gekennzeichnet, dass** in dem ersten Schritt (1) der Seheindruck der Person mittels einer Wellenfrontfehlermessung und/oder einer Messung einer subjektiven Refraktion bestimmt wird.

8. Verfahren nach einem der Ansprüche 1 bis 7, **dadurch gekennzeichnet, dass** in dem zweiten Schritt (2) der Seheindruck der Person durch eine Wellenfrontfehlermessung bestimmt wird.

## Claims

1. Method for determining correction characteristics of a visual aid for at least one eye of a person, an individual physiology of the person being taken into account in the processing of the retinal image in order to attain an individual optimum of visual comfort for the person, wherein:
- in a first step (1), a visual impression of the person is determined under a first usage condition, after which
- in a second step (2), a visual impression of the person is determined under a second usage condition, and after which
- in a third step (3), the wavefront of the person is optimized by determining the correction characteristics of the visual aid until the visual impression of the person under the second usage condition at least approximately matches the visual impression of the person under the first usage condition was determined in the first step (1).

2. Method according to Claim 1, **characterized in that** the first usage condition is in the light.

3. Method according to Claim 1 or 2, **characterized in that** the second usage condition is in the dark.

4. Method according to Claim 1, 2 or 3, **characterized in that** further metrics are used in parallel to determine the visual impression.

5. Method according to Claim 4, **characterized in that** a point spread function, a contrast vision and/or a dynamic vision are used as further metrics.

6. Method according to one of Claims 1 to 5, **characterized in that** the visual aid is a spectacle lens.

7. Method according to one of Claims 1 to 6, **characterized in that**, in the first step (1) the visual impression of the person is determined by means of a wavefront error measurement and/or a measurement of a subjective refraction.

8. Method according to one of Claims 1 to 7, **characterized in that**, in the second step (2), the visual impression of the person is determined by a wavefront error measurement.

## Revendications

1. Procédé de détermination de propriétés correctrices d'une aide visuelle pour au moins un oeil d'une personne, une physiologie individuelle de la personne étant prise en compte lors du traitement de l'image visuelle rétinienne en vue d'obtenir un optimum individuel en matière de confort visuel pour la personne, procédé selon lequel :
- dans une première étape (1), une impression visuelle de la personne est déterminée sous une première condition d'utilisation, après quoi
- dans une deuxième étape (2), une impression visuelle de la personne est déterminée sous une deuxième condition d'utilisation, et après quoi
- dans une troisième étape (3), le front d'onde de la personne est optimisé par la détermination des propriétés correctrices de l'aide visuelle jusqu'à ce que l'impression visuelle de la personne sous la deuxième condition d'utilisation coïncide avec l'impression visuelle de la personne sous la première condition d'utilisation déterminée dans la première étape (1).

2. Procédé selon la revendication 1, **caractérisé en ce que** la première condition d'utilisation est dans la lumière.

3. Procédé selon la revendication 1 ou 2, **caractérisé en ce que** la deuxième condition d'utilisation est dans l'obscurité.

4. Procédé selon la revendication 1, 2 ou 3, **caractérisé en ce que** des indices de mesure supplémentaires sont utilisés en parallèle pour déterminer l'impression visuelle.

5. Procédé selon la revendication 4, **caractérisé en ce que** les indices de mesure supplémentaires utilisés sont une fonction d'étalement du point, une vision du contraste et/ou une vision dynamique.

6. Procédé selon l'une des revendications 1 à 5, **caractérisé en ce que** l'aide visuelle est un verre de lunette.

7. Procédé selon l'une des revendications 1 à 6, **caractérisé en ce que** dans la première étape (1), l'impression visuelle de la personne est déterminée au moyen d'une mesure de l'erreur de front d'onde et/ou d'une mesure d'une réfraction subjective.

8. Procédé selon l'une des revendications 1 à 7, **caractérisé en ce que** dans la deuxième étape (2), l'impression visuelle de la personne est déterminée par le biais d'une mesure de l'erreur de front d'onde.
